# EUROPÄISCHE PATENTSCHRIFT

(11) **EP 0 794 945 B1**
(45) Veröffentlichungstag und Bekanntmachung des Hinweises auf die Patenterteilung: **12.09.2001**
(21) Anmeldenummer: 95938452.0
(22) Anmeldetag: 20.11.1995
(51) Int. Cl.: C07D 239/26, C07D 239/34, C07D 213/53, A01N 43/54, A01N 43/40

(54) **IMINOOXYBENZYLCROTONSÄUREESTER, VERFAHREN ZU IHRER HERSTELLUNG UND IHRE VERWENDUNG**
IMINOOXYBENZYLCROTONIC ACID ESTERS, PROCESS FOR THEIR PREPARATION AND USE THEREOF
ESTERS D'ACIDE D'IMMINO-OXYBENZYLCROTONIQUE, LEURS PROCEDES DE PREPARATION ET LEUR UTILISATION

(30) Priorität: 30.11.1994 DE 4442560
(43) Veröffentlichungstag der Anmeldung: 17.09.1997
(73) Patentinhaber: BASF AKTIENGESELLSCHAFT, 67056 Ludwigshafen (DE)
(72) Erfinder: MÜLLER, Bernd, D-67227 Frankenthal (DE); GRAMMENOS, Wassilios, D-67063 Ludwigshafen (DE); SAUTER, Hubert, D-68167 Mannheim (DE); RÖHL, Franz, D-67105 Schifferstadt (DE); AMMERMANN, Eberhard, D-64646 Heppenheim (DE); LORENZ, Gisela, D-67434 Hambach (DE); GÖTZ, Norbert, D-67547 Worms (DE)
(74) Vertreter: Fitzner, Ulrich, Dr.
(86) Internationale Anmeldenummer: EP9504555
(87) Internationale Veröffentlichungsnummer: WO9616943

(56) Entgegenhaltungen:
- EP-A- 0 463 488
- EP-A- 0 513 580
- WO-A-94/08968

## Beschreibung

Die vorliegende Erfindung betrifft Iminooxybenzylcrotonsäureester der Formel I 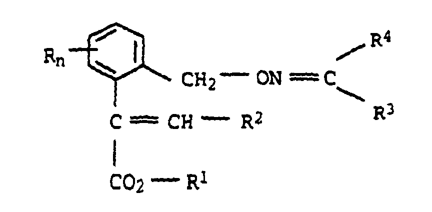 in denen der Index und die Substituenten die folgende Bedeutung haben:
- n: 0, 1, 2, 3 oder 4, wobei die Substituenten R verschieden sein können, wenn n größer als 1 ist;
- R: Nitro, Cyano, Halogen,
ggf. subst. Alkyl, Alkenyl, Alkinyl, Alkoxy, Alkenyloxy, Alkinyloxy oder
für den Fall, daß n für 2 steht zusätzlich eine an zwei benachbarte Ringatome gebundene ggf. subst. Brücke, welche drei bis vier Glieder aus der Gruppe 3 oder 4 Kohlenstoffatome, 2 oder 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann;
- R¹: C₁-C₄-Alkyl;
- R²: Wasserstoff oder C₁-C₄-Alkyl;
- R³: Wasserstoff, Cyano, Halogen, Alkyl, Halogenalkyl, Alkoxy, Halogenalkoxy, Alkylthio, Halogenalkylthio oder Cycloalkyl;
- R⁴: ein 5-10-gliedriges mono- oder bicyclisches, heteroaromatisches Ringsystem, welches durch übliche Gruppen substituiert ist,
sowie Verfahren zu ihrer Herstellung und ihre Verwendung.

Aus der Literatur sind 2-Phenylcrotonsäuren mit fungizider Wirksamkeit bekannt (EP-A 280 185; EP-A 463 488; DE-A 41 16 090).

Der vorliegenden Erfindung lagen Wirkstoffe mit verbesserter Wirksamkeit als Aufgabe zugrunde.

Demgemäß wurden die eingangs definierten Verbindungen I gefunden. Außerdem wurden Verfahren zu ihrer Herstellung, sie enthaltende Mittel und Verfahren zu ihrer Verwendung insbesondere zur Bekämpfung von tierischen Schädlingen und Schadpilzen gefunden.

Die Verbindunge I sind nach verschiedenen Methoden erhältlich. Besonders vorteilhaft erhält man sie dadurch, daß man ein Benzylderivat der Formel II mit einem Oxim-der Formel III oder dessen Salz umsetzt. 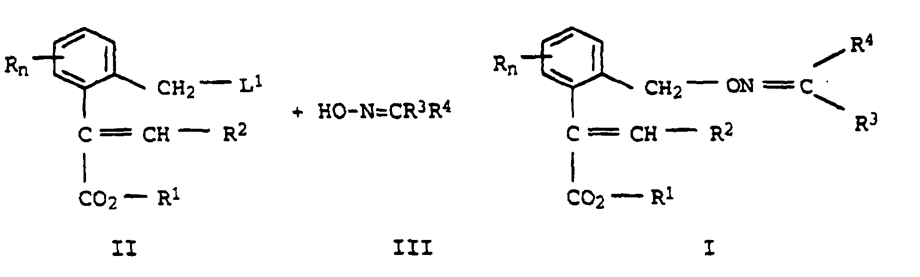 L¹ in der Formel II steht für eine Abgangsgruppe, d.h. eine nucleophil austauschbare Gruppe wie Halogen (z.B. Chlor, Brom und Iod), oder ein Alkyl oder Arylsulfonat (z.B. Methylsulfonat, Trifluormethylsulfonat, Phenylsulfonat und 4-Methylphenylsulfonat).

Die Oxime III können auch in Form ihrer Salze, z.B. mit anorganischen Säuren wie Hydrochloride, Hydrobromide, Hydrosulfate und Hydrophosponate, verwendet werden.

Die Umsetzung der Verbindungen II und III erfolgt üblicherweise bei Temperaturen von 0°C bis 80°C, vorzugsweise 20°C bis 60°C, in einem inerten Lösungsmittel in Gegenwart einer Base.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Dieethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton, Toluol, tert.-Butylmethylether und Dimethylform-amid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxy-magnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Kaliumcarbonat und Kalium-tert.-butanolat. Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.

Die für die Herstellung der Verbindungen I benötigten Ausgangsstoffe II sind aus EP-A 463 488 bekannt oder können nach den dort beschriebenen Methoden hergestellt werden.

Die Oxime III und ihre Salze sind aus der Literatur (WO-A 92/13,830, WO-A 92/18,487 und WO-A 94/08,968) bekannt oder können nach den dort beschriebenen Methoden hergestellt werden.

Es kann für die Umsetzung vorteilhaft sein, zunächst das Oxim III bzw. dessen Salz mit der Base in die entsprechende Base zu überführen, welche dann mit dem Benzylderivat II umgesetzt wird.

Daneben erhält man die Verbindungen I durch Umsetzung von α-Ketoestern IV im Sinne einer Wittig-Reaktion.

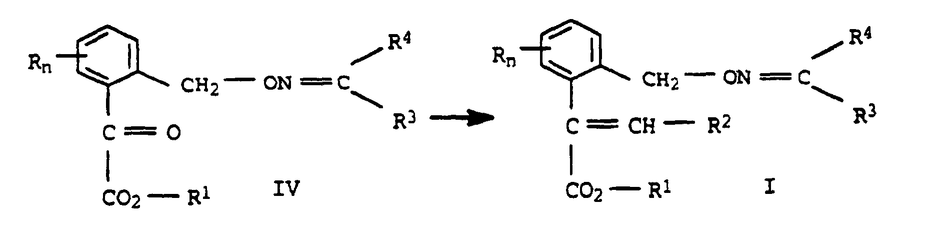

Die α-Ketoester IV sind nach bekannten Methoden [z.B. EP-A 493 711; Synthetic Commun. 11, 943 (1981)] erhältlich.

Die Verbindungen I können saure oder basische Zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schweferlsäure, Salpetersäure), organsiche Säuren (z.B. Ameisensäure, Essigsäure, Oxalsäure, Malonsäure, Milchsäure, Äpfelsäure, Bernseinsäure, Weinsäure, Zitronensäure, Salizylsäure, p-Toluolsulfonsäure, Dodecylbenzolsulfonsäure) oder andere protonenacide Verbindungen (z.B. Saccharin).

Basen für Basenadditionsprodukte sind u.a. Oxide, Hydroxide, Carbonate oder Hydrogencarbonate von Alkalimetallen oder Erdalkaimetallen (z.B. Kalium- oder Natriumhydroxyd oder -carbonat) oder Ammoniumverbindungen (z.B. Ammoniumhydroxyd).

Die Verbindungen I können aufgrund ihrer Doppelbindungen in verschiedenen Raumformen (Isomeren) gebildet und verwendet werden.

Nach den bisherigen Erkenntnissen sind im Hinblick auf die Anwendung besonders solche Verbindungen I bevorzugt, in denen die "Crotonester"-Doppelbindung E-konfiguriert ist und/oder in denen R³ und der Sauerstoff auf der gleichen Seite der C=N-Doppelbindung stehen.

Bei den in den vorstehenden Formeln angegebenen Definitionen der Symbole wurden z.T. Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit vorzugsweise 1 bis 10 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit vorzugsweise 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit vorzugsweise 1 bis 10 Kohlenstoffatomen (wie vorstehend genannt), welche über ein Sauerstoffatom (-O-) an das Gerüst gebunden sind;
Halogenalkoxy: geradkettige oder verzweigte Halogenalkylgruppen mit vorzugsweise 1 bis 4 Kohlenstoffatomen (wie vorstehend genannt), welche über ein Sauerstoffatom (-O-) an das Gerüst gebunden sind;
Alkylthio: geradkettige oder verzweigte Alkylgruppen mit vorzugsweise 1 bis 4 Kohlenstoffatomen (wie vorstehend genannt), welche über ein Schwefelatom (-S-) an das Gerüst gebunden sind;
Halogenalkylthio: geradkettige oder verzweigte Halogenalkylgruppen mit vorzugsweise 1 bis 4 Kohlenstoffatomen (wie vorstehend genannt), welche über ein Schwefelatom (-S-) an das Gerüst gebunden sind;
Alkenyl: ungesättigte, geradkettige oder verzweigte Kohlenwasserstoffreste mit vorzugsweise 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkenyloxy: geradkettige oder verzweigte Alkenylgruppen mit vorzugsweise 3 bis 10 Kohlenstoffatomen (wie vorstehend genannt), welche über ein Sauerstoffatom (-O-) an das Gerüst gebunden sind;
Alkinyl: geradkettige oder verzweigte Kohlenwasserstoffgruppen mit vorzugsweise 2 bis 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Alkinyloxy: geradkettige oder verzweigte Alkinylgruppen mit vorzugsweise 3 bis 10 Kohlenstoffatomen (wie vorstehend genannt), welche über ein Sauerstoffatom (-O-) an das Gerüst gebunden sind;
Cycloalkyl: mono- oder bicyclische Kohlenwaserstoffreste mit vorzugsweise 3 bis 10 Kohlenstoffatomen, z.B. C₃-C₁₀-(Bi)cycloalkyl wie Cyclopropyl, Cyclobutyl, Cyclopentyl, Cyclohexyl, Cycloheptyl, Bornanyl, Norbornanyl, Dicyclohexyl, Bicyclo[3,3,0]octyl, Bicyclo[3,2,1]octyl, Bicyclo[2,2,2]octyl oder Bicyclo[3,3,1]nonyl;
eine an zwei benachbarte Ringatome gebundene Brücke, welche drei bis vier Glieder aus der Gruppe 3 oder 4 Kohlenstoffatome, 2 oder 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann: Brücken, die mit dem Ring, an den sie gebunden sind beispielsweise eines der folgenden Systeme bilden: Chinolinyl, Benzofuranyl und Naphthyl;
ein 5-10 gliedriges mono- oder bicyclisches, heteroaromatisches Ringsystem: 5- oder 6-gliedrige Heteroaromaten welche zusätzlich benzokondensiert oder an ein weiteres 5- oder 6-gliedriges heteroaromatisches Ringsystem kondensiert sein können, z.B.
   - **5-gliedriges Heteroaryl, enthaltend ein bis vier Stickstoffatome oder ein bis drei Stickstoffatome und ein Schwefel-oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol -3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - **benzo- oder heterokondensiertes 5-gliedriges Heteroaryl, enthaltend ein bis drei Stickstoffatome oder ein Stickstoffatom und ein Sauerstoff- oder Schwefelatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe oder eine 3- bis 4-gliedrige ungesättigte Kette, welche neben Kohlenstoffgliedern beispielsweise Stickstoffatome und/oder ein Sauerstoff- oder Schwefelatom enthalten kann, verbrückt sein können, z.B. Benzimidazolyl, Benzthiazolyl und Benzoxyzolyl;
   - **6-gliedriges Heteroaryl, enthaltend ein bis drei bzw. ein bis vier Stickstoffatome:** 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
   - **benso- oder heterokondensiertes 6-gliedriges Heteroaryl, enthaltend ein bis drei bzw. ein bis vier Stickstoffatome und/ oder ein Sauerstoff- oder Schwefelatom:** 6-Ring Heteroarylgruppen, welche neben Kohlenstoffatomen ein bis drei Stickstoffatome als Ringglieder enthalten können, und in welchen zwei benachbarte Kohlenstoffringglieder oder ein Stickstoff-und ein benachbartes Kohlenstoffringglied durch eine Buta-1,3-dien-1,4-diylgruppe oder eine 3- bis 4-gliedrige ungesättigte Kette, welche neben Kohlenstoffgliedern beispielsweise Stickstoffatome und/oder ein Sauerstoff- oder Schwefelatom enthalten kann, verbrückt sein können, z.B. Indolyl, Chnolinyl und Isochinolinyl.

Der Zusatz "*ggf. subst.*" in Bezug auf *Alkyl-, Alkenyl- und Alkinylgruppen* soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor oder Brom) ersetzt sein können und/ oder einen bis drei (vorzugsweise einen) der folgenden Reste tragen können:
Cyano, Nitro, Hydroxy, Amino, Formyl, Carboxyl, Aminocarbonyl, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten;
unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten.

Der Zusatz *"ggf. subst"* bzw. in Bezug auf die *cyclischen (gesättigten, ungesättigten oder aromatischen) Gruppen* soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor oder Brom, insbesondere Fluor oder Chlor) ersetzt sein können und/oder einen bis vier (insbesondere einen bis drei) der folgenden Reste
Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
und/oder einen bis drei (insbesondere einen) der folgenden Reste
unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten,
und oder einen oder zwei (insbesondere einen) der folgenden Reste
Formyl oder CRⁱⁱⁱ=NOR^{iv}, wobei Rⁱⁱⁱ Wasserstoff oder Alkyl und R^{iv} Alkyl, Alkenyl, Alkinyl und Arylalkyl bedeutet und wobei die genannten Alkylgruppen vorzugsweise 1 bis 6 Kohlenstoffatome, insbesondere 1 bis 4 Kohlenstoffatome, enthalten; die genannten Alkenyl- bzw. Alkinylgruppen enthalten vorzugsweise 2 bis 6 Kohlenstoffatome, insbesondere 3 bis 6 Kohlenstoffatome und Aryl bedeutet insbesondere Phenyl, welches unsubstituiert ist oder durch übliche Gruppen substituiert sein kann,
tragen kann oder bei denen zwei benachbarte C-Atome der cyclischen Systeme eine C₃-C₅-Alkylen-, C₃-C₅-Alkenylen-, Oxy-C₂-C₄-alkylen-, Oxy-C₁-C₃-alkylenoxy, Oxy-C₂-C₄-alkenylen-, Oxy-C₂-C₄-alkenylenoxy- oder Butadiendiylgruppe tragen können, wobei diese Brücken ihrerseits partiell oder vollständig halogeniert sein können und/oder einen bis drei, insbesondere einen oder zwei der folgenden Reste tragen können: C₁-C₄-Alkyl, C₁-C₄-Halogenalkyl, C₁-C₄-Alkoxy, C₁-C₄-Halogenalkoxy und C₁-C₄-Alkylthio.

Unter *"üblichen Gruppen*", die als Substituenten von R⁴ in Betracht kommen, sind die vorstehend als mögliche Substituenten von cyclischen Systemen genannten Reste zu verstehen.

Im Hinblick auf ihre biologische Wirkung sind Verbindungen I be- . vorzugt, in denen n für 0 oder 1, insbesondere 0, steht.

Für den Fall, daß n für 1 steht, werden Verbindungen I bevorzugt, in denen R für eine der folgenden Gruppen steht:
Halogen (insbesondere Fluor und Chlor), Methyl, Trifluormethyl, Methoxy und Cyano.

Außerdem werden Verbindungen I, in denen n für 1 steht, bevorzugt, in denen R in 3- oder 6-Position zum Crotonester steht.

Außerdem werden Verbindungen I besonders bevorzugt, in denen R¹ für Methyl steht.

Gleichermaßen besonders bevorzugt sind Verbindungen I, in denen R¹ für Ethyl steht.

Daneben werden Verbindungen I bevorzugt, in denen R² für Wasserstoff steht.

Daneben werden Verbindungen I bevorzugt, in denen R² für Methyl steht.

Außerdem werden Verbindungen I besonders bevorzugt, in denen R² für Ethyl steht.

Daneben werden Verbindungen I bevorzugt, in denen R³ für C₁-C₄-Akly (insbesondere Methyl) steht.

Außerdem werden Verbindungen I besonders bevorzugt, in denen R³ für Halogen (insbesondere Chlor und Brom), C₁-C₄-Alkoxy und C₁-C₄-Alkylthio steht.

Gleichermaßen besonders bevorzugt sind Verbindungen I, in denen R³ für Cyclopropyl steht.

Daneben werden Verbindungen I besonders bevorzugt, in denen R³ für substituiertes Trifluormethyl steht.

Daneben werden Verbindungen I bevorzugt, in denen R⁴ für substituiertes 2-, 3- oder 4-Pyridinyl steht.

Außerdem werden Verbindungen I besonders bevorzugt, in denen R⁴ für substituiertes 3- oder 4-Pyridazinyl steht.

Gleichermaßen besonders bevorzugt sind Verbindungen I, in denen R⁴ für substituiertes 2-, 4- oder 5 Pyrimidinyl steht.

Daneben werden Verbindungen I besonders bevorzugt, in denen R⁴ für substituiertes Pyrazinyl oder Triazinyl steht.

Daneben werden Verbindungen I bevorzugt, in denen R⁴ durch Halogen, Alkyl, Halogenalkyl, Alkoxy, Halogenalkoxy, Alkylthio und Halogenalkylthio substituiert ist, wobei diese Reste ihrerseits weitere übliche Substituenten tragen können.

Außerdem werden Verbindungen I besonders bevorzugt, in denen R⁴ durch Aryl, Aryloxy, Hetaryl und Hetaryloxy substituiert ist, wobei diese Reste ihrerseits weitere übliche Substituenten tragen können.

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinarsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis. Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor; aus der Ordnung der Pflanzensauger (Homoptera) beispielsweise Acyrthosiphon onöbrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus; Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus Christel, Trichodorus viruliferus, Xiphinema index, Xiphinema mediterraneum.

Die Verbindungen I können als solche, in Form ihrer Formulierungen oder den daraus bereiteten Anwendungsformen, z.B. in Form von direkt versprühbaren Lösungen, Pulvern, Suspensionen oder Dispersionen, Emulsionen, öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz z.B. zum Schutz von Holz, Papier und Textilien eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse-Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsaure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-form-aldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen betragt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Emährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thio-carbamoyl)-disulfid; Nitroderivate, wie Dinitro-(1-methyl-heptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyll-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylaminobenzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethylfuran-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesaure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl-(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsaureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

### Beispiel 1

### α-(2-(4'-Ethoxy-pyrimidin-2'-yl-methyliminoxymethyl)-phenyl)-crotonsäuremethylester

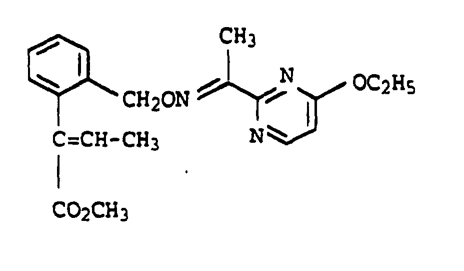

Eine Mischung von 2,0 g (7,4 mmol) α-(2-Brommethylphenyl)-crotonsäuremethylester (DE 41 16 090), 1,35 g (7,4 mmol) 2-Acetyl-4-ethoxy-pyrimidin-oxim (WO 92/18487) und 1,5 g (11 mmol) K₂CO₃ in 10 ml Dimethylformamid wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden einmal mit Wasser extrahiert, über MgSO₄ getrocknet und eingeengt. Der Rückstand wird säulenchromatographisch mit Cyclohexan/Essigester-Gemischen gereinigt. Man erhält 1,6 g (58 %) der Titelverbindung als hellgelbes Öl.
¹H-NMR (CDCl₃; δ in ppm):
8,5 (d, 1H, Pyrimidinyl); 7,55 (m, 1H, Phenyl); 7,3 (m, 3H, 2XPhenyl, =CH-); 7,1 (m, 1H, Phenyl); 6,65 (d, 1H, Pyrimidinyl); 5,25 (s, breit, OCH₂): 4,5 (q, 2H, OCH₂); 3,7 (s, 3H, OCH₃); 2,3 (s, 3H, CH₃); 1,6 (d, 3H, CH₃); 1,4 (t, 3H, CH₃)

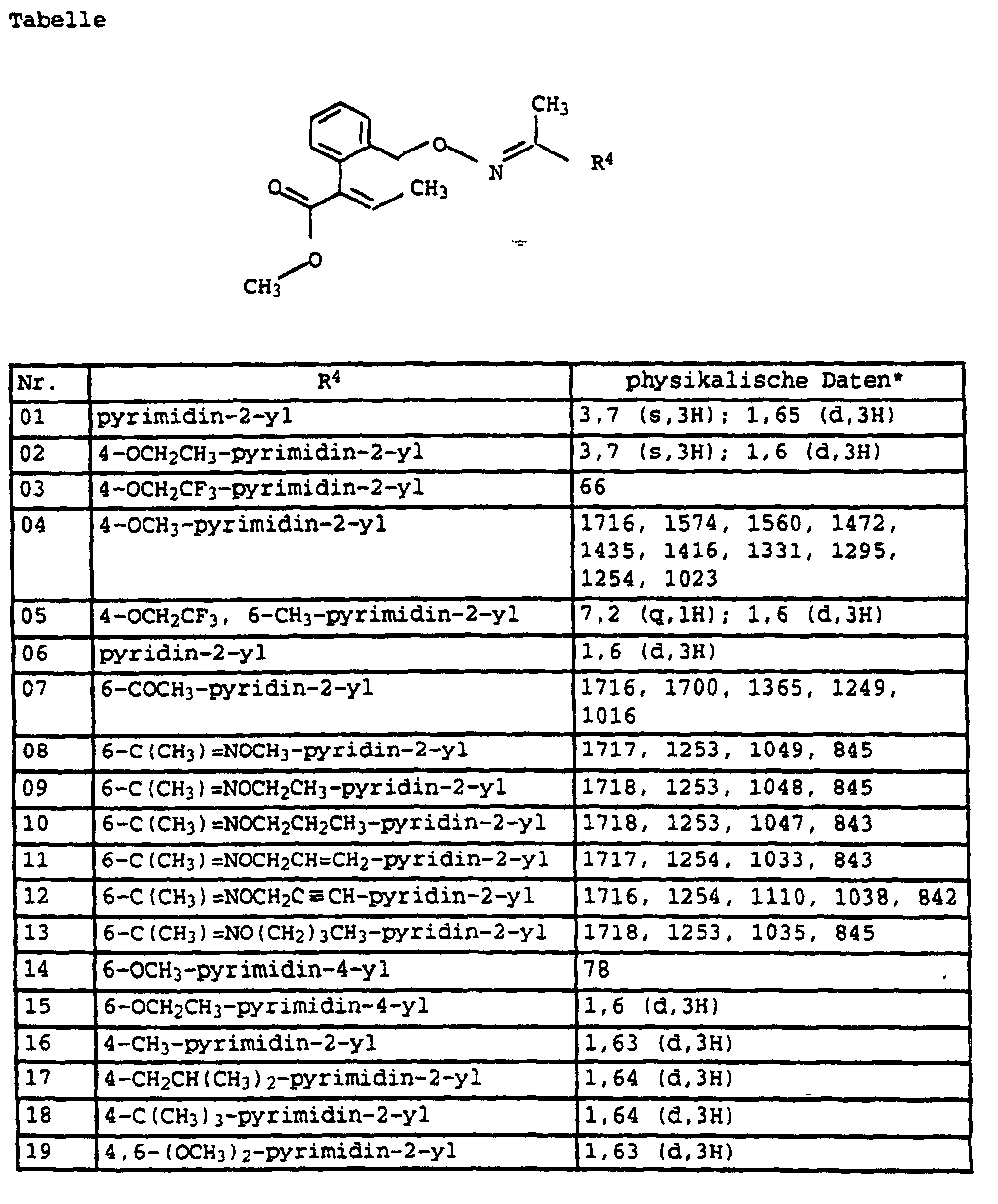

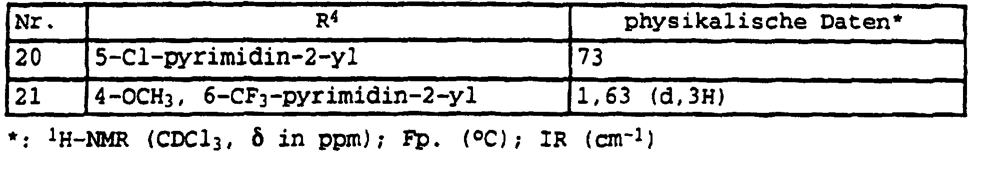

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Puccinia recondita (Weizenbraunrost)

Blätter von Weizensämlingen (Sorte "Kanzler") wurden mit Sporen des Braunrosts (Puccinia recondita) bestäubt. Die so behandelten Pflanzen wurden 24 h bei 20-22°C und einer relativen Luftfeuchtigkeit von 90-95 % inkubiert und anschließend mit der wäßrigen Wirkstoffaufbereitung behandelt. Nach weiteren 8 Tagen bei 20-22°C und 65-70 % relativer Luftfeuchtigkeit wurde das Ausmaß der Pilzentwicklung ermittelt. Die Auswertung erfolgte visuell.

In diesem Test zeigten die mit den Verbindungen 1, 2, 3 und 4 behandelten Pflanzen einen Befall von 15 % und weniger, während die unbehandelten Pflanzen zu 70 % befallen waren.

### Pyricularia oryzae (Reisbrand)

Reiskeimlinge (Sorte "Tain Nong 67") wurden mit der Wirkstoffaufbereitung tropfnaß gespritzt. Nach 24 Stunden wurden die Pflanzen mit einer wäßrigen Sporensuspension des Pilzes Pyricularia oryzae besprüht und 6 Tage bei 22-24°C bei einer relativen Luftfeuchtigkeit von 95-99 % bewahrt. Die Beurteilung erfolgte visuell.

In diesem Test zeigten die mit den Verbindungen 1, 2,3 und 4 behandelten Pflanzen einen Befall von 15 % und weniger, während die unbehandelten Pflanzen zu 60 % befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Iminooxybenzylcrotonsäureester der Formel I 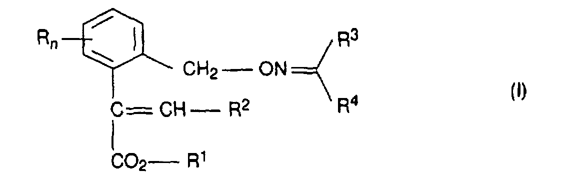 in denen der Index und die Substituenten die folgende Bedeutung haben:
n 0, 1, 2, 3 oder 4, wobei die Substituenten R verschieden sein können, wenn n größer als 1 ist;
R Nitro, Cyano, Halogen,
C₁-C₁₀-Alkyl, C₂-C₁₀-Alkenyl, C₂-C₁₀-Alkinyl, C₁-C₁₀-Alkoxy, C₃-C₁₀-Alkenyloxy und C₃-C₁₀-Alkinyloxy, wobei diese Gruppen partiell oder vollständig halogeniert sein können und/oder einen bis drei der folgenden Reste tragen können:
• Cyano, Nitro, Hydroxy, Amino, Formyl, Carboxyl, Aminocarbonyl,
• C₁-C₆-Alkoxy, C₁-C₆-Halogenalkoxy, C₁-C₆-Alkylthio, C₁-C₆-Halogenalkylthio, C₁-C₆-Alkylamino, Di-C₁-C₆-alkylamino, C₁-C₆-Alkylcarbonyl, C₁-C₆-Alkoxycarbonyl, C₁-C₆-Alkylcarbonyloxy, C₁-C₆-Alkylaminocarbonyl, Di-C₁-C₆-alkylaminocarbonyl, C₁-C₆-Alkylcarbonylamino, C₁-C₆-Alkoxycarbonylamino und C₁-C₆-Alkylcarbonyl-N-C₁-C₆-alkylamino,
• unsubstituiertes oder durch übliche Gruppen substituiertes C₃-C₁₂-Cycloalkyl, C₃-C₁₂-Cycloalkoxy, C₃-C₁₂-Cycloalkylthio, C₃-C₁₂-Cycloalkylamino, C₃-C₁₂-Cycloalkyl-N-C₁-C₆-alkylamino,
• unsubstituiertes oder durch übliche Gruppen substituiertes 3- bis 12-gliedriges Heterocyclyl, Heterocyclyloxy, Heterocyclylthio, Heterocyclylamino oder Heterocyclyl-N-C₁-C₆-alkylamino,
• unsubstituiertes oder durch übliche Gruppen substituiertes Aryl, Aryloxy, Arylthio, Arylamino, Aryl-N-C₁-C₆-alkylamino, Aryl-C₁-C₆-alkoxyl Aryl-C₁-C₆-alkylthio, Aryl-C₁-C₆-alkylamino, Aryl-C₁-C₆-alkyl-N-C₁-C₆-alkylamino, Hetaryl, Hetaryloxy, Hetarylthio, Hetarylamino, Hetaryl-N-C₁-C₆-alkylamino, Hetaryl-C₁-C₆-alkoxy, Hetaryl-C₁-C₆-alkylthio, Hetaryl-C₁-C₆-alkylamino und Hetaryl-C₁-C₆-alkyl-N-C₁-C₆-alkylamino,
oder
für den Fall, daß n für 2 steht, zusätzlich eine an zwei benachbarte Ringatome gebundene ggf. subst. Brücke, welche drei bis vier Glieder aus der Gruppe 3 oder 4 Kohlenstoffatome, 2 oder 3 Kohlenstoffatome und 1 oder 2 Stickstoff-, Sauerstoff- und/oder Schwefelatome enthält, wobei diese Brücke gemeinsam mit dem Ring, an den sie gebunden ist, einen partiell ungesättigten oder aromatischen Rest bilden kann;
R¹ C₁-C₄-Alkyl;
R² Wasserstoff oder C₁-C₄-Alkyl;
R³ Wasserstoff, Cyano, Halogen, C₁-C₁₀-Alkyl, C₁-C₄-Halogenalkyl, C₁-C₁₀-Alkoxy, C₁-C₄-Halogenalkoxy, C₁-C₄-Alkylthio, C₁-C₄-Halogenalkylthio oder C₃-C₁₀-Cycloalkyl;
R⁴ 5-gliedriges Heteroaryl, enthaltend ein bis vier Stickstoffatome oder ein bis drei Stickstoffatome und ein Schwefel- oder Sauerstoffatom oder
6-gliedriges Heteroaryl, enthaltend ein bis drei bzw. ein bis vier Stickstoffatome,
wobei die cyclischen Gruppen partiell oder vollständig halogeniert sein können und/oder einen bis vier der folgenden Reste
Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
und oder einen oder zwei (insbesondere einen) der folgenden Reste
Formyl oder CRⁱⁱⁱ=NOR^{iv}, wobei Rⁱⁱⁱ Wasserstoff oder Alkyl und R^{iv} Alkyl, Alkenyl, Alkinyl und Arylalkyl bedeutet und wobei die genannten Alkylgruppen vorzugsweise 1 bis 6 Kohlenstoffatome, insbesondere 1 bis 4 Kohlenstoffatome, enthalten; die genannten Alkenyl- bzw. Alkinylgruppen enthalten vorzugsweise 2 bis 6 Kohlenstoffatome, insbesondere 3 bis 6 Kohlenstoffatome und Aryl bedeutet insbesondere Phenyl, welches unsubstituiert ist oder durch folgende Gruppen substituiert sein kann:
Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
und oder einen oder zwei (insbesondere einen) der folgenden Reste
Formyl oder CRⁱⁱⁱ=NOR^{iv}, wobei Rⁱⁱⁱ Wasserstoff oder Alkyl und R^{iv} Alkyl, Alkenyl, Alkinyl und Arylalkyl bedeutet und wobei die genannten Alkylgruppen vorzugsweise 1 bis 6 Kohlenstoffatome, insbesondere 1 bis 4 Kohlenstoffatome, enthalten; die genannten Alkenyl- bzw. Alkinylgruppen enthalten vorzugsweise 2 bis 6 Kohlenstoffatome, insbesondere 3 bis 6 Kohlenstoffatome und Aryl bedeutet insbesondere Phenyl.

2. Verbindungen der Formel I gemäß Anspruch 1, in denen n für 0 oder 1 steht.

3. Verbindungen der Formel I gemäß Anspruch 1, in denen R für Halogen, Methyl, Trifluormethyl, Methoxy und Cyano steht.

4. Verbindungen der Formel I gemäß Anspruch 1, in denen R⁴ für Pyridinyl, Pyridazinyl, Pyrimidinyl, Pyrazinyl oder Triazinyl steht, welches gemäß Anspruch 1 substituiert ist.

5. Verfahren zur Herstellung der Verbindungen der Formel I gemäß Anspruch 1, **dadurch gekennzeichnet, daß** man ein Benzylderivat der Formel II 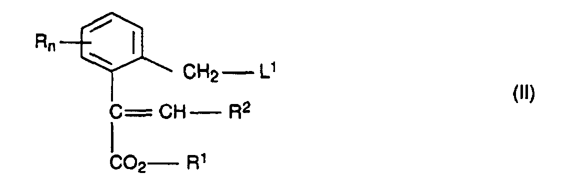 in der L¹ für eine Abgangsgruppe steht, mot einem Oxim der Formel III 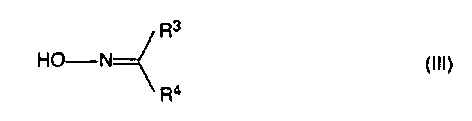 oder dessen Salz umsetzt.

6. Zur Bekämpfung von tierischen Schädlingen oder Schadpilzen geeignetes Mittel, enthaltend eine festen oder flüssigen Trägerstoff und eine Verbindung der Formel I gemäß Anspruch 1.

7. Verwendung der Verbindungen I gemäß Anspruch 1 zur Herstellung eines zur Bekämpfung von tierischen Schädlingen oder Schadpilzen geeigneten Mittels.

8. Verfahren zur Bekämpfung von Schadpilzen, **dadurch gekennzeichnet, daß** man die Pilze oder die vor Pilzbefall zu schützenden Materialien, Pflanzen, Boden oder Saatgüter mit einer wirksamen Menge einer Verbindung der Formel I gemäß Anspruch 1 behandelt.

9. Verfahren zur Bekämpfung von tierischen Schädlingen, **dadurch gekennzeichnet, daß** man die Schädlinge oder die vor ihnen zu schützenden Materialien, Pflanzen, Boden oder Saatgüter mit einer wirsamen Menge einer Verbindung der Formel I gemäß Anspruch 1 behandelt.

## Claims

1. An iminooxybenzylcrotonate ester of the formula I 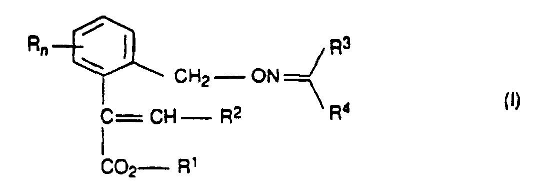 where the index and the substituents have the following meanings:
n is 0, 1, 2, 3 or 4, it being possible for the substituents R to be different if n is greater than 1;
R is nitro, cyano, halogen,
C₁-C₁₀-alkyl, C₂-C₁₀-alkenyl, C₂-C₁₀-alkynyl, C₁-C₁₀-alkoxy, C₃-C₁₀-alkenyloxy and C₃-C₁₀-alkynyloxy, where these groups can be partially or completely halogenated and/or can carry one to three of the following radicals:
• cyano, nitro, hydroxyl, amino, formyl, carboxyl, aminocarbonyl,
• C₁-C₆-alkoxy, C₁-C₆-haloalkoxy, C₁-C₆-alkylthio, C₁-C₆-haloalkylthio, C₁-C₆-alkylamino, di-C₁-C₆-alkylamino, C₁-C₆-alkylcarbonyl, C₁-C₆-alkoxycarbonyl, C₁-C₆-alkylcarbonyloxy, C₁-C₆-alkylaminocarbonyl, di-C₁-C₆-alkylaminocarbonyl, C₁-C₆-alkylcarbonylamino, C₁-C₆-alkoxycarbonylamino and C₁-C₆-alkylcarbonyl-N-C₁-C₆-alkylamino,
• C₃-C₁₂-cycloalkyl, C₃-C₁₂-cycloalkoxy, C₃-C₁₂-cycloalkylthio, C₃-C₁₂-cycloalkylamino, C₃-C₁₂-cycloalkyl-N-C₁-C₆-alkylamino which is unsubstituted or substituted by customary groups,
• 3- to 12-membered heterocyclyl, heterocyclyloxy, heterocyclylthio, heterocyclylamino or heterocyclyl-N-C₁-C₆-alkylamino unsubstituted or substituted by customary groups,
• aryl, aryloxy, arylthio, arylamino, aryl-N-C₁-C₆-alkylamino, aryl-C₁-C₆-alkoxy, aryl-C₁-C₆-alkylthio, aryl-C₁-C₆-alkylamino, aryl-C₁-C₆-alkyl-N-C₁-C₆-alkylamino, hetaryl, hetaryloxy, hetarylthio, hetarylamino, hetaryl-N-C₁-C₆-alkylamino, hetaryl-C₁-C₆-alkoxy, hetaryl-C₁-C₆-alkylthio, hetaryl-C₁-C₆-alkylamino and hetaryl-C₁-C₆-alkyl-N-C₁-C₆-alkylamino which is unsubstituted or substituted by customary groups,
or
in the case where n is 2, additionally an unsubst. or subst. bridge bonded to two adjacent ring atoms, which contains three to four members from the group consisting of 3 or 4 carbon atoms, 2 or 3 carbon atoms and 1 or 2 nitrogen, oxygen and/or sulfur atoms, it being possible for this bridge, together with the ring to which it is bonded, to form a partially unsaturated or aromatic radical;
R¹ is C₁-C₄-alkyl;
R² is hydrogen or C₁-C₄-alkyl;
R³ is hydrogen, cyano, halogen, C₁-C₁₀-alkyl, C₁-C₄-haloalkyl, C₁-C₁₀-alkoxy, C₁-C₄-haloalkoxy, C₁-C₄-alkylthio, C₁-C₄-haloalkylthio or C₃-C₁₀-cycloalkyl;
R⁴ is 5-membered heteroaryl containing one to four nitrogen atoms or one to three nitrogen atoms and one sulfur or oxygen atom or
is 6-membered heteroaryl containing one to three or one to four nitrogen atoms,
where the cyclic groups can be partially or completely halogenated, and/or contain one to four of the following radicals
cyano, nitro, hydroxyl, amino, carboxyl, aminocarbonyl, alkyl, haloalkyl, alkenyl, haloalkenyl, alkenyloxy, haloalkenyloxy, alkynyl, haloalkynyl, alkynyloxy, haloalkynyloxy, alkoxy, haloalkoxy, alkylthio, haloalkylthio, alkylamino, dialkylamino, alkylcarbonyl, alkoxycarbonyl, alkylcarbonyloxy, alkylaminocarbonyl, dialkylaminocarbonyl, alkylcarbonylamino, alkoxycarbonylamino, alkylcarbonyl-N-alkylamino and alkylcarbonyl-N-alkylamino, the alkyl groups in these radicals preferably containing 1 to 6 carbon atoms, in particular 1 to 4 carbon atoms and said alkenyl or alkynyl groups in these radicals containing 2 to 8, preferably 2 to 6, in particular 2 to 4, carbon atoms;
and/or one or two (in particular one) of the following radicals
formyl or CRⁱⁱⁱ=NOR^{iv}, Rⁱⁱⁱ being hydrogen or alkyl and R^{iv} being alkyl, alkenyl, alkynyl or arylalkyl and said alkyl groups preferably containing 1 to 6 carbon atoms, in particular 1 to 4 carbon atoms; said alkenyl or alkynyl groups preferably contain 2 to 6 carbon atoms, in particular 3 to 6 carbon atoms, and aryl is in particular phenyl, which is unsubstituted or can be substituted by the following groups:
cyano, nitro, hydroxyl, amino, carboxyl, aminocarbonyl, alkyl, haloalkyl, alkenyl, haloalkenyl, alkenyloxy, haloalkenyloxy, alkynyl, haloalkynyl, alkynyloxy, haloalkynyloxy, alkoxy, haloalkoxy, alkylthio, haloalkylthio, alkylamino, dialkylamino, alkylcarbonyl, alkoxycarbonyl, alkylcarbonyloxy, alkylaminocarbonyl, dialkylaminocarbonyl, alkylcarbonylamino, alkoxycarbonylamino, alkylcarbonyl-N-alkylamino and alkylcarbonyl-N-alkylamino, the alkyl groups in these radicals preferably containing 1 to 6 carbon atoms, in particular 1 to 4 carbon atoms and said alkenyl or alkynyl groups in these radicals containing 2 to 8, preferably 2 to 6, in particular 2 to 4, carbon atoms;
and/or one or two (in particular one) of the following radicals
formyl or CRⁱⁱⁱ=NOR^{iv}, Rⁱⁱⁱ being hydrogen or alkyl and R^{iv} being alkyl, alkenyl, alkynyl or arylalkyl and said alkyl groups preferably containing 1 to 6 carbon atoms, in particular 1 to 4 carbon atoms; said alkenyl or alkynyl groups preferably contain 2 to 6 carbon atoms, in particular 3 to 6 carbon atoms, and aryl is in particular phenyl.

2. A compound of the formula I as claimed in claim 1, in which n is 0 or 1.

3. A compound of the formula I as claimed in claim 1, in which R is halogen, methyl, trifluoromethyl, methoxy or cyano.

4. A compound of the formula I as claimed in claim 1, in which R⁴ is pyridinyl, pyridazinyl, pyrimidinyl, pyrazinyl or triazinyl which is substituted as set forth in claim 1.

5. A process for preparing the compounds of the formula I as claimed in claim 1, which comprises reacting a benzyl derivative of the formula II 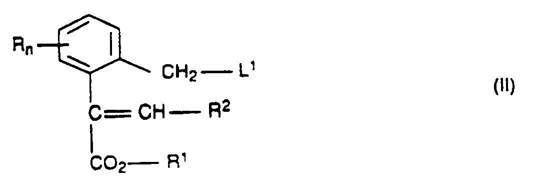 where L¹ is a leaving group, with an oxime of the formula III 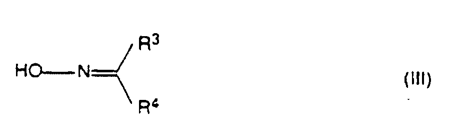 or its salt.

6. A composition suitable for controlling animal pests or harmful fungi, containing a solid or liquid carrier and a compound of the formula I as claimed in claim 1.

7. The use of the compounds I as claimed in claim 1 for preparing a composition suitable for controlling animal pests or harmful fungi.

8. A method of controlling harmful fungi, which comprises treating the fungi or the materials, plants, soil or seed to be protected from fungal attack with an active amount of a compound of the formula I as claimed in claim 1.

9. A method of controlling animal pests, which comprises treating the pests or the materials, plants, soil or seed to be protected from them with an active amount of a compound of the formula I as claimed in claim 1.

## Revendications

1. Esters d'acide iminooxybenzylcrotonique de formule I 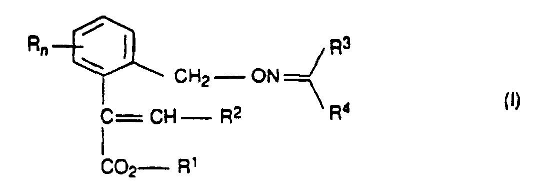 dans lesquels l'indice et les substituants ont la signification suivante:
n 0, 1, 2, 3 ou 4, tandis que les substituants R peuvent être différents, lorsque n est supérieur à 1;
R nitro, cyano, halogéno,
alkyle en C₁-C₁₀, alcényle en C₂-C₁₀, alcynyle en C₂-C₁₀, alcoxy en C₁-C₁₀, alcényloxy en C₃-C₁₀ et alcynyloxy en C₃-C₁₀, tandis que ces groupes peuvent être partiellement ou totalement halogénés et/ou peuvent porter un à trois des restes suivants:
• cyano, nito, hydroxy, amino, formyle, carboxyle, aminocarbonyle,
• alcoxy en C₁-C₆, halogénoalcoxy en C₁-C₆, alkyl(C₁-C₆)thio, halogénoalkyl(C₁-C₆)thio, alkyl(C₁-C₆)amino, dialkyl(C₁-C₆)amino, alkyl-(C₁-C₆)carbonyle, alcoxy(C₁-C₆)carbonyle, alkyl(C₁-C₆)carbonyloxy, alkyl(C₁-C₆)amino-carbonyle, dialkyl(C₁-C₆)aminocarbonyle, alkyl-(C₁-C₆)carbonylamino, alcoxy(C₁-C₆)carbonyl-amino et alkyl(C₁-C₆)carbonyl-N-alkyl(C₁-C₆)-amino,
• cycloalkyle en C₃-C₁₂, cycloalcoxy en C₃-C₁₂, cycloalkylthio en C₃-C₁₂, cycloalkylamino en C₃-C₁₂, cycloalkyl(C₃-C₁₂)-N-alkylamino en C₁-C₆, non-substitué ou substitué par des groupes classiques,
• hétérocyclyle, hétérocyclyloxy, hétérocyclyl-thio, hétérocyclylamino ou hétérocyclyl-N-alkyl(C₁-C₆)amino à 3 à 12 chaînons, non-substitué ou substitué par des groupes classiques,
• aryle, aryloxy, arylthio, arylamino, aryl-N-alkyl(C₁-C₆)amino, arylalcoxy(C₁-C₆), aryl-alkyl(C₁-C₆)thio, aryl-alkyl(C₁-C₆)amino, aryl-alkyl-(C₁-C₆)-N-alkyl(C₁-C₆)amino, hétaryle, hétaryloxy, hétarylthio, hétaryl-amino, hétaryl-N-alkyl(C₁-C₆)-amino, hétarylalcoxy-(C₁-C₆), hétaryl-alkyl-(C₁-C₆)thio, hétaryl-alkyl(C₁-C₆)amino et hétaryl-alkyl-(C₁-C₆)-N-alkyl(C₁-C₆)amino, non-substitué ou substitué par des groupes classiques,
ou
dans le cas où n vaut 2, en outre un pont relié à deux atomes cycliques contigus, éventuellement substitué, qui contient trois à quatre membres du groupe de 3 ou 4 atomes de carbone, 2 ou 3 atomes de carbone et 1 ou 2 atomes d'azote, d'oxygène et/ou de soufre, tandis que ce pont peut former, conjointement avec le cycle auquel il est relié, un reste partiellement insaturé ou aromatique;
R¹ alkyle en C₁-C₄;
R² hydrogène ou alkyle en C₁-C₄;
R³ hydrogène, cyano, halogéno, alkyle en C₁-C₁₀, halogénoalkyle en C₁-C₄, alcoxy en C₁-C₁₀, halogénoalcoxy en C₁-C₄, alkyl(C₁-C₄)thio, halogénoalkyl(C₁-C₄)thio ou cycloalkyle en C₃-C₁₀;
R⁴ hétéroaryle à 5 chaînons, contenant un à quatre atomes d'azote, ou un à trois atomes d'azote et un atome de soufre ou d'oxygène, ou
hétéroaryle à 6 chaînons, contenant un à trois ou un à quatre atomes d'azote,
tandis que les groupes cycliques peuvent être partiellement ou totalement halogénés et/ou contiennent un à quatre des restes suivants
cyano, nitro, hydroxy, amino, carboxyle, aminocarbonyle, alkyle, halogénoalkyle, alcényle, halogénoalcényle, alcényloxy, halogénoalcényloxy, alcynyle, halogéno-alcynyle, alcynyloxy, halogénoalcynyloxy, alcoxy, halogénoalcoxy, alkylthio, halogénoalkylthio, alkylamino, dialkylamino, alkyl-carbonyle, alcoxycarbonyle, alkylcarbonyloxy, alkylaminocarbonyle, dialkylaminocarbonyle, alkylcarbonylamino, alcoxycarbonylamino, alkylcarbonyl-N-alkylamino et alkylcarbonyl-N-alkylamino, tandis que les groupes alkyle dans ces restes contiennent de préférence 1 à 6 atomes de carbone, en particulier 1 à 4 atomes de carbone, et que les groupes alcényle ou alcynyle mentionnés dans ces restes contiennent 2 à 8, de préférence 2 à 6, en particulier 2 à 4 atomes de carbone;
et/ou un ou deux (en particulier un) des restes suivants
formyle ou CRⁱⁱⁱ=NOR^{iv}, tandis que Rⁱⁱⁱ représente l'hydrogène ou un groupe alkyle et R^{iv} un groupe alkyle, alcényle, alcynyle et arylalkyle, et tandis que les groupes alkyle mentionnés présentent de préférence 1 à 6 atomes de carbone, en particulier 1 à 4 atomes de carbone; que les groupes alcényle et alcynyle mentionnés contiennent de préférence 2 à 6 atomes de carbone, en particulier 3 à 6 atomes de carbone et aryle signifie en particulier un groupe phényle, qui peut être non-substitué ou substitué par les groupes suivants:
cyano, nitro, hydroxy, amino, carboxyle, aminocarbonyle, alkyle, halogénoalkyle, alcényle, halogénoalcényle, alcényloxy, halogénoalcényloxy, alcynyle, halogéno-alcynyle, alcynyloxy, halogénoalcynyloxy, alcoxy, halogénoalcoxy, alkylthio, halogéno-alkylthio, alkylamino, dialkylamino, alkyl-carbonyle, alcoxycarbonyle, alkylcarbonyloxy, alkylaminocarbonyle, dialkylaminocarbonyle, alkylcarbonylamino, alcoxycarbonylamino, alkylcarbonyl-N-alkylamino et alkylcarbonyl-N-alkylamino, tandis que les groupes alkyle dans ces restes contiennent de préférence 1 à 6 atomes de carbone, en particulier 1 à 4 atomes de carbone, et que les groupes alcényle ou alcynyle mentionnés dans ces restes contiennent 2 à 8, de préférence 2 à 6, en particulier 2 à 4 atomes de carbone;
et/ou un ou deux (de préférence un) des restes suivants formyle ou CRⁱⁱⁱ=NOR^{iv}, tandis que Rⁱⁱⁱ représente l'hydrogène ou un groupe alkyle et R^{iv} un groupe alkyle, alcényle, alcynyle et arylalkyle, et tandis que les groupes alkyle mentionnés présentent de préférence 1 à 6 atomes de carbone, en particulier 1 à 4 atomes de carbone; que les groupes alcényle et alcynyle mentionnés contiennent de préférence 2 à 6 atomes de carbone, en particulier 3 à 6 atomes de carbone et aryle signifie en particulier un groupe phényle.

2. Composés de formule I selon la revendication 1, dans lesquels n vaut 0 ou 1.

3. Composés de formule I selon la revendication 1, dans lesquels R désigne un groupe halogéno, méthyle, trifluorométhyle, méthoxy et cyano.

4. Composés de formule I selon la revendication 1, dans lesquels R⁴ désigne un groupe pyridinyle, pyridazinyle, pyrimidinyle, pyrazinyle ou triazinyle, qui est substitué selon la revendication 1.

5. Procédé pour la préparation des composés de formule I selon la revendication 1, **caractérisé par** le fait qu'on fait réagir un dérivé benzylique de formule II 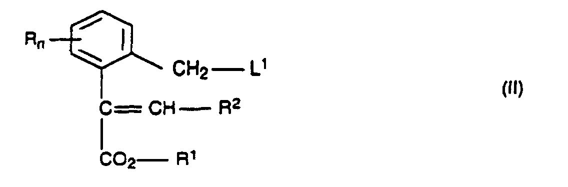 dans laquelle L¹ désigne un groupe séparable, avec une oxime de formule III 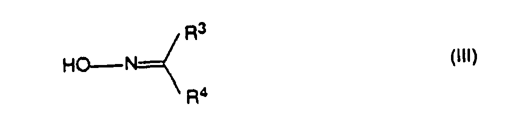 ou un sel de celle-ci.

6. Produit approprié pour la lutte contre les parasites animaux ou les champignons nuisibles, contenant un support solide ou liquide et un composé de formule I selon la revendication 1.

7. Utilisation de composés I selon la revendication 1 pour la préparation d'un produit pour la lutte contre des parasites animaux ou des champignons nuisibles.

8. Procédé pour la lutte contre des champignons nuisibles, **caractérisé par** le fait qu'on traite les champignons ou les matières, plantes, sols ou semences à protéger contre l'invasion de champignons, avec une quantité efficace d'un composé de formule I selon la revendication 1.

9. Procédé pour la lutte contre des parasites animaux, **caractérisé par** le fait qu'on traite les parasites ou les matières, plantes, sols ou semences à protéger contre eux, avec une quantité efficace d'un composé de formule I selon la revendication 1.
